# EUROPEAN PATENT SPECIFICATION

(11) **EP 1 885 857 B1**
(45) Date of publication and mention of the grant of the patent: **27.02.2013**
(21) Application number: 06768510.7
(22) Date of filing: 17.05.2006
(51) Int. Cl.: C12N 15/29

(54) **CELLS OR PLANTS HAVING A PRODUCING ABILITY OF POLYLACTATE OR ITS COPOLYMERS AND METHOD FOR PREPARING POLYLACTATE OR ITS COPOLYMERS USING THE SAME**
ZELLEN ODER PFLANZEN MIT DER FÄHIGKEIT ZUR PRODUKTION VON POLYLACTAT ODER SEINEN COPOLYMEREN SOWIE VERFAHREN ZUR HERSTELLUNG VON POLYLACTAT ODER SEINEN COPOLYMEREN UNTER VERWENDUNG DAVON
CELLULES OU PLANTES POSSÉANT LA CAPACITÉ DE PRODUCTION DU POLYLACTATE OU SES COPOLYMÈRES ET PROCÉDÉ DE PRÉPARATION DU POLYLACTATE OU SES COPOLYMÈRES LES UTILISANT

(30) Priority: 24.05.2005 KR 20050043798
(43) Date of publication of application: 13.02.2008
(73) Proprietor: LG Chem, Ltd., Seoul 150-721 (KR); Korea Advanced Institute of Science and Technology, 305-701 Daejeon (KR)
(72) Inventor: Cho, Jun-Hyeong, Yuseong-gu, Daejeon 305-762 (KR); Park, Si-Jae, Seo-gu, Daejeon 302-740 (KR); Lee, Sang-Yup, Yuseong-gu, Daejeon 305-762 (KR); Jung, Yu-Kyung, Yeseong-gu, Daejeon 305-701 (KR)
(74) Representative: Cabinet Plasseraud
(86) International application number: PCT/KR2006/001845
(87) International publication number: WO 2006/126796

(56) References cited:
- WO-A2-2004/024876
- ABE H ET AL: "Physical properties and enzymatic degradability of copolymers of (R)-3-hydroxybutyric acid and (S,S)-lactide" POLYMER, ELSEVIER SCIENCE PUBLISHERS B.V, GB, vol. 39, no. 1, 1 January 1998 (1998-01-01), pages 59-67, XP004096154 ISSN: 0032-3861
- NAWRATH C ET AL: "PLASTID TARGETING OF THE ENZYMES REQUIRED FOR THE PRODUCTION OF POLYHYDROXYBUTYRATE IN HIGHER PLANTS" STUDIES IN POLYMER SCIENCE, ELSEVIER SCIENCE PUBLISHERS B.V., AMSTERDAM, NL, vol. 12, 1 January 1994 (1994-01-01), pages 136-149, XP000564151 ISSN: 0922-5579
- MOIRE L ET AL: "SYNTHESIS OF NOVEL BIOMATERIALS IN PLANTS" JOURNAL OF PLANT PHYSIOLOGY, FISCHER, STUTTGART, DE, vol. 160, no. 7, 1 July 2003 (2003-07-01), pages 831-839, XP008046688 ISSN: 0176-1617
- BONGCAM V ET AL: "Strange plastic-producing plants" BIOFUTUR, LAVOISIER, CACHAN, FR, vol. 1998, no. 184, 1 December 1998 (1998-12-01), pages 84-85, XP004280614 ISSN: 0294-3506
- S. TAGUCHI ET AL: "A microbiol factory for lactate based polyesters using a lactate-polymerizing enzyme" P.N.A.S., vol. 105, no. 45, 11 November 2008 (2008-11-11), pages 17323-17327, XP002524123
- PARK S.Y.: 'Biosynthesis of poly(3-hydroxybutylrate-co-3-hydroxyalkanoa tes) by metabolically engineered Escherichia coli strains' APPLIED BIOCHEMISTRY AND BIOTECHNOLOGY vol. 114, 2004, pages 335 - 346, XP008073097
- ZHANG S.: 'Comparative study of the relationship between monomer structure and reactivity for two polyhydroxyalkanoate synthases' APPLIED MICROBIOLOGY AND BIOTECHNOLOGY vol. 56, no. 1-2, July 2001, pages 131 - 136, XP003004146
- TAKAGI M.: 'Effect of poly DL-lactic-co-glycolic acid mesh on a three-dimensional culture of chondrocytes' JOURNAL OF BIOSCIENCE AND BIOENGINEERING vol. 98, no. 6, 2004, pages 477 - 481, XP004727094

## Description

### TECHNICAL FIELD

The present invention relates to cells or plants having the ability of producing polylactate or its copolymers and a method for preparing polylactate or its copolymers using the same, more specifically, relates to cells or plants capable of expressing a gene encoding an enzyme converting lactate into lactyl-CoA and a gene encoding an enzyme involved in PHA synthesis and a method for preparing polylactate or hydroxyalkanoate-lactate copolymer, the method comprises: culturing the cells in a medium containing lactate, carbon sources providing hydroxyalkanoyl-CoA or lactate and various hydroxyalkanoate, or culturing the plants.

### BACKGROUND ART

Polylactate(PLA) is a typical biodegradable polymer originated from lactate, which has a variety of applications as a common or a medical polymer. At present, PLA is being prepared by polymerizing lactate which is produced by fermenting microorganisms, but only low molecular weight PLA(1000-5000 dalton) is produced by direct polymerization of lactate. To synthesize high molecular weight(>100,000 dalton) of PLA, a method polymerizing low molecular weight PLA obtained by direct polymerization of lactate using a chain coupling agent to obtain higher molecular weight PLA can be used. But it has disadvantages in that, the process for preparing PLA of high molecular weight is complicated due to the addition of a solvent or a chain coupling agent and also it isn't easy to remove them. At present, in the process for preparing commercially available PLA of high molecular weight, a method, in which lactate is converted into lactide to synthesize PLA by cyclodehydration of the lactide ring, is being used.

Meanwhile, PHA is a polyester which microorganisms accumulate therein as a carbon and energy storage compound when other nutritive elements lack(for example, phosphorus, nitrogen, magnesium, oxygen) while there are excessive carbon source. PHA is recognized as an alternative material for existing synthesized plastics since it has a similar property to synthesized polymers originated from existing petroleum, at the same time, shows an excellent biodegradation rate.

The existing PHA is divided into SCL-PHA(short-chain-length PHA) having short carbon chains and MCL-PHA(medium-chain-length PHA) having long carbon chains. A gene synthesizing PHA was cloned from *Ralstonia eutropha, Pseudomonas* and PHA consisting of various monomers was synthesized by recombinant microorganisms *(*Qi et al., FEMS Microbiol. Lett., 157:155, 1997; Qi et al., FEMS Microbiol. Lett., 167:89, 1998; Langenbach et al., FEMS Microbiol. Lett., 150:303, 1997; WO 01/55436; US 6,143,952; WO 98/54329; WO 99/61624).

To produce PHA in microorganisms, an enzyme which converts metabolites of the microorganisms into a PHA monomer, and PHA synthase which synthesizes PHA polymer using PHA monomer are required. PHA synthase synthesizes PHA using hydroxyacyl-CoA as a substrate and β-ketothiolase(*PhaA*), acetoacetyl-CoA reductase(*PhaB*), cloned from *Ralstonia eutropha* etc., 3-hydroxydecanoyl-ACP:CoA transferase(*PhaG*) cloned from *Pseudomonas,* (R)-specific enoyl-CoA hydratase(*PhaJ*) derived from *Aeromonas caviae* and *Pseudomona aeruginosa* (Fukui et al., J. Bacteriol., 180:667, 1998; Tsage et al., FEMS Microbiol. Lett., 184:193, 2000), 3-ketoacyl-ACP reductase(*FabG*) derived from *E*. *coli* and *Pseudomonas aeruginosa* (Taguchi et al., FEMS Microbiol. Lett., 176:183, 1999; Ren et al., J. Bacteriol., 182:2978, 2000; Park et al., FEMS Microbiol. Lett., 214:217, 2002) are known as enzymes capable of providing hydroxyacyl-CoA which is a substrate of PHA. Various kinds of PHAs have been synthesized by hydroxyalkanoate hydroxylated at various positions in the carbon chain(mainly the 3, 4, 5, 6 positions) using these enzymes.

However, it is reported that it has little PHA synthase activity on hydroxyalkanoate hydroxylated at the 2-position (Zhang et al., Appl. Microbiol. Biotechnol., 56:131, 2001; Valentin and Steinbuchel, Appl. Microbiol. Biotechnol., 40:699, 1994; Yuan et al., Arch. Biochem. Biophyics., 394:87, 2001). Until now, PHA synthase activity on lactyl-CoA by in vitro activity measurement of PHA polymerase on lactyl-CoA has been reported, but PHA synthase activity on lactyl-CoA is very weak (Zhang et al., Appl. Microbiol. Biotechnol., 56:131, 2001; Valentin and Steinbuchel, Appl. Microbiol. Biotechnol., 40:699,1994). That is, there are no examples that PHA and its copolymers are produced naturally or using recombinant cells or plants because hydroalkanoate, such as lactate hydroxylated at the 2-carbon position isn't suitable for substrate specificity of PHA synthase.

Therefore, the present inventors have made extensive efforts to produce high molecular weight PLA and its copolymers using cells or plants, as a result, confirmed that poly[hydroxyalkanoate-co-lactate] copolymers were produced by culturing recombinant *Ralstonia eutropha* transformed into propionyl-CoA transferase gene(pct) derived from *Clostridium propionicum* which is a gene encoding an enzyme converting lactate into lactyl-CoA in a production medium containing lactate and PLA was produced by culturing recombinant *E.coli* transformed into PHA synthase gene derived from *Bacillus cereus* and *pct* gene in a production medium containing lactate, thereby completing the present invention.

### DISCLOSURE OF THE INVENTION

The main object of the present invention is to provide transformed cells having the ability of producing polylactate or hydroxyalkanoate-lactate copolymer [poly(hydroxyalkanoate-co-lactate)], which contains a gene encoding propionyl-CoA transferase and a gene encoding polyhydroxyalkanoates synthase selected from phaC and phaRBC, wherein said transformed cells are obtained by transforming cells without having a gene encoding polyhydroxyalkanoates synthase selected from phaC and phaRBC with a recombinant vector containing a gene encoding propionyl-CoA transferase and a gene encoding polyhydroxyalkanoates synthase selected from phaC and phaRBC.

Another object of the present invention is to provide transformed cells having the ability of producing polylactate or hydroxyalkanoate-lactate copolymer [poly(hydroxyalkanoate-co-lactate)], which contains a gene encoding propionyl-CoA transferase and a gene encoding polyhydroxyalkanoates synthase selected from phaC and phaRBC,
wherein said transformed cells are obtained :
➢ by transforming cells having a gene encoding polyhydroxyalkanoates synthase selected from phaC and phaRBC with a recombinant vector containing a gene encoding propionyl-CoA transferase; or
➢ by transforming cells having a gene encoding propionyl-CoA transferase with a recombinant vector containing a gene encoding polyhydroxyalkanoates synthase selected from phaC and phaRBC.

Another object of the present invention is to provide a method for preparing polylactate or its copolymers by culturing said cells or plants.

To achieve the above object, in one aspect, the present invention provides cells or plants having the ability of producing polylactate or hydroxyalkanoate-lactate copolymers[poly(hydroxyalkanoate-co-lactate)], which simultaneously have a gene encoding an enzyme converting lactate into lactyl-CoA and a gene encoding polyhydroxyalkanoates(PHA) synthase capable of using lactyl-CoA as a substrate.

In another aspect, the present invention provides a method for preparing polylactate or hydroxyalkanoate-lactate copolymers[poly(hydroxyalkanoate-co-lactate)], the method comprising the steps of culturing the cells in a medium containing lactate or lactate and hydroxyalkanoate as a carbon source; and recovering polylactate or hydroxyalkanoate-lactate copolymers[poly(hydroxyalkanoate-co-lactate)] from the cultured cells or culturing the plants; and recovering polylactate or hydroxyalkanoate-lactate copolymer from the cultured plants.

In still another aspect, the present invention provides a recombinant vector for preparing polylactate or hydroxyalkanoate-lactate copolymers[poly(hydroxyalkanoate-co-lactate)] which contains a gene encoding an enzyme converting lactate into lactyl-CoA and a gene encoding PHA synthase capable of using lactyl-CoA as a substrate, and cells or plants transformed with the recombinant vector.

In yet another aspect, the present invention provides a method for preparing polylactate or hydroxyalkanoate-lactate copolymers[poly(hydroxyalkanoate-co-lactate)], the method comprising the steps of culturing the transformed cells in a medium containing lactate or hydroxyalkanoate and lactate as a carbon source; and recovering polylactate or hydroxyalkanoate-lactate copolymers from the cultured cells or culturing the transformed plants; and recovering polylactate or hydroxyalkanoate-lactate copolymer from the cultured plants.

In yet another aspect, the present invention provides hydroxyalkanoate-lactate copolymers[poly(hydroxyalkanoate-co-lactate)], containing, as a monomer, lactate and one or more hydroxyalkanoate selected from the group consisting of 3-hydroxybutyrate, 3-hydroxyvalerate, 4-hydroxybutyrate, (D)-3-hydroxycarboxylic acids of the medium chain length(C6-14), 3-hydroxypropionic acid, 3-hydroxyhexanoic acid, 3-hydroxyheptanoic acid, 3-hydroxyoctanoic acid, 3-hydroxynonanoic acid, 3-hydroxydecanoic acid, 3-hydroxyundecanoic acid, 3-hydroxydodecanoic acid, 3-hydroxytetradecanoic acid, 3-hydroxyhexadecanoic acid, 4-hydroxyvaleric acid, 4-hydroxyhexanoic acid, 4-hydroxyheptanoic acid, 4-hydroxyoctanoic acid, 4-hydroxydecanoic acid, 5-hydroxyvaleric acid, 5-hydroxyhexanoic acid, 6-hydroxydodecanoic acid, 3-hydroxy-4-pentenoic acid, 3-hydroxy-4-trans-hexenoic acid, 3-hydroxy-4-cis-hexenoic acid, 3-hydroxy-5-hexenoic.acid, 3-hydroxy-6-trans-octenoic acid, 3-hydroxy-6-cis-octenoic acid, 3-hydroxy-7-octenoic acid, 3-hydroxy-8-nonenoic acid, 3-hydroxy-9-decenoic acid, 3-hydroxy-5-cis-dodecenoic acid, 3-hydroxy-6-cis-dodecenoic acid, 3-hydroxy-5-cis-tetradecenoic acid, 3-hydroxy-7-cis-tetradecenoic acid, 3-hydroxy-5,8-cis-cis-tetradecenoic acid, 3-hydroxy-4-methylvaleric acid, 3-hydroxy-4-methyhexanoic acid, 3-hydroxy-5-methylhexanoic acid, 3-hydroxy-6-methylheptanoic acid, 3-hydroxy-4-methyloctanoic acid, 3-hydroxy-5-methyloctanoic acid, 3-hydroxy-6-methyloctanoic acid, 3-hydroxy-7-methyloctanoic acid, 3-hydroxy-6-methylnonanoic acid, 3-hydroxy-7-methylnonanoic acid, 3-hydroxy-8-methylnonanoic acid, 3-hydroxy-7-methyldecanoic acid, 3-hydroxy-9-methyldecanoic acid, 3-hydroxy-7-methyl-6-octenoic acid, malic acid, 3-hydroxysuccinic acid-methylester, 3-hydroxyadipinic acid-methylester, 3-hydroxysuberic acid-methylester, 3-hydroxysebacic acid-methylester, 3-hydroxyazelaic acid-methylester, 3-hydroxysuberic acid-ethylester, 3-hydroxysebacic acid-ethylester, 3-hydroxypimelic acid-prophylester, 3-hydroxysebacic acid-benzylester, 3-hydroxy-8-acetoxyoctanoic acid, 3-hydroxy-9-acetoxynonanoic acid, phenoxy-3-hydroxybutyric acid, phenoxy-3-hydroxyvaleric acid, phenoxy-3-hydroxyheptanoic acid, phenoxy-3-hydroxyoctanoic acid, para-cyanophenoxy-3-hydroxybutyric acid, para-cyanophenoxy-3-hydroxyvaleric acid, para-cyanophenoxy-3-hydroxyhexanoic acid, para-nitrophenoxy-3-hydroxyhexanoic acid, 3-hydroxy-5-phenylvaleric acid, 3-hydroxy-5-cyclohexylbutyric acid, 3,12-dihydroxydodecanoic acid, 3,8-dihydroxy-5-cis-tetradecenoic acid, 3-hydroxy-4,5-epoxydecanoic acid, 3-hydroxy-6,7-epoxydodecanoic acid, 3-hydroxy-8,9-epoxy-5,6-cis-tetradecanoic acid, 7-cyano-3-hydroxyheptanoic acid, 9-cyano-3-hydroxynonanoic acid, 3-hydroxy-7-fluoroheptanoic acid, 3-hydroxy-9-fluorononanoic acid, 3-hydroxy-6-chlorohexanoic acid, 3-hydroxy-8-chlorooctanoic acid, 3-hydroxy-6-bromohexanoic acid, 3-hydroxy-8-bromooctanoic acid, 3-hydroxy-11-bromoundecanoic acid, 3-hydroxy-2-butenoic acid, 6-hydroxy-3-dodecenoic acid, 3-hydroxy-2-methylbutyric acid, 3-hydroxy-2-methylvaleric acid and 3-hydroxy-2,6-dimethyl-5-heptenoic acid.

In yet another aspect, the present invention provides 3HA having three to twelve carbons and lactate copolymer[poly(3HA-co-lactate)].

Other features and embodiments of the present invention will be more fully apparent from the following detailed description and appended claims.

### BRIEF DESCRIPTION OF DRAWINGS

FIG. 1 is a gene map of recombinant plasmid pTac99Pct containing *pct* derived from *Clostridium propionicum.*
FIG. 2 is a gene map of recombinant plasmid pMCSTac99Pct containing *pct* derived from *Clostridium propionicum.*
FIG. 3 is a gene map of recombinant plasmid p10499BcRBC containing *phaRBC* derived from *Bacillus cereus.*
FIG. 4 is a gene map of recombinant plasmid p10499613C2 *containing phaC2_{PS},* a gene encoding PHA synthase derived from *Pseudomonas* sp. 61-3.
FIG. 5 is a schematic diagram showing a pathway for synthesizing P(3HB-co-lactate) using glucose and lactate.
FIG. 6 is a schematic diagram showing a pathway for synthesizing copolymer P(3HB-co-4HB-co-lactate) using glucose, 4HB, 3HP and lactate.
FIG. 7 is a schematic diagram showing a pathway for synthesizing PLA using cells.
FIG. 8 is a schematic diagram showing a pathway for synthesizing copolymers P(3HA-co-lactate) containing medium chain length 3-hydroxyalkanoate(3HA) having three to twelve carbons and lactate as a monomer.
FIG. 9 is ¹H-NMR result of P(3HB-co-LA) prepared from *R. eutropha* NCIMB 11599(pMCSTacPct).
FIG. 10 is ¹³C-NMR result of P(3HB-co-LA) prepared from *R. eutropha* NCIMB 11599(pMCSTacPct).
FIG. 11 is ¹H-NMR result of PLA prepared from *E*. *coli* XL1-Blue(p10499BcRBC + pMCSTacPct).

### DETAILED DESCRIPTION OF THE INVENTION AND PREFERRED EMBODIMENTS

The present invention, in one aspect, relates to transformed cells having the ability of producing polylactate or hydroxyalkanoate-lactate copolymer [poly(hydroxyalkanoate-co-lactate)], which contains a gene encoding propionyl-CoA transferase and a gene encoding polyhydroxyalkanoates synthase selected from phaC and phaRBC, wherein said transformed cells are obtained by transforming cells without having a gene encoding polyhydroxyalkanoates synthase selected from phaC and phaRBC with a recombinant vector containing a gene encoding propionyl-CoA transferase and a gene encoding polyhydroxyalkanoates synthase selected from phaC and phaRBC.

The present invention also relates to transformed cells having the ability of producing polylactate or hydroxyalkanoate-lactate copolymer [poly(hydroxyalkanoate-co-lactate)], which contains a gene encoding propionyl-CoA transferase and a gene encoding polyhydroxyalkanoates synthase selected from phaC and phaRBC,
wherein said transformed cells are obtained :
➢ by transforming cells having a gene encoding polyhydroxyalkanoates synthase selected from phaC and phaRBC with a recombinant vector containing a gene encoding propionyl-CoA transferase; or
➢ by transforming cells having a gene encoding propionyl-CoA transferase with a recombinant vector containing a gene encoding polyhydroxyalkanoates synthase selected from phaC and phaRBC.

The cells or plants having the ability of producing polylactate or hydroxyalkanoate-lactate copolymers[poly(hydroxyalkanoate-co-lactate)] (i) are obtained by transforming cells or plants without having a gene encoding PHA synthase with a gene encoding an enzyme converting lactate into lactyl-CoA and a gene encoding PHA synthase capable of using lactyl-CoA as a substrate, or (ii) are obtained by transforming cells or plants having a gene encoding PHA synthase using lactyl-CoA as a substrate with a gene encoding enzyme converting lactate into lactyl-CoA, or (iii) are obtained by transforming cells or plants having a gene encoding an enzyme converting lactate into lactyl-CoA with a gene encoding PHA synthase capable of using lactyl-CoA as a substrate, but it isn't limited thereto. For example, in a cell having any one gene of both genes(a gene encoding PHA synthase capable of using lactyl-CoA as a substrate and a gene encoding an enzyme converting lactate into lactyl-CoA), the gene amplified and transformed with the other gene is also included in the scope of the present invention.

In the present invention, a gene encoding an enzyme converting lactate into lactyl-CoA is preferably propionyl-CoA transferase(*pct*). The inventive cells or plants are preferably transformed with a recombinant vector containing *pct* gene, at the same time, transformed with a vector containing *phaRBC* or *phaRBC* is preferably inserted onto chromosome thereof. In this case, if lactate or hydroxyalkanoate and lactate are used as a carbon source, it is possible to prepare polylactate or hydroxyalkanoate-lactate copolymers[poly(hydroxyalkanoate-co-lactate)]. Furthermore, a gene encoding PHA synthase capable of using lactyl-CoA as a substrate is preferably *phaC.* The inventive cells or plants are preferably transformed with a recombinant vector containing *pct* gene, at the same time, transformed with a vector containing *phac* or *phaC* is preferably inserted onto chromosome thereof. In this case, if fatty acid and lactate are used as a carbon source, it is possible to prepare a copolymer[poly(3HA-co-lactate)] of 3HA having three to twelve carbons and lactate.

Various microorganisms are known as cells having a gene encoding PHA synthase (KR 10-250830 B1). For example, there are microorganisms, including microorganisms of the genus *Achromobacter* containing *Achromobacter* sp., *Achromobacter xylosoxidans,* etc., microorganisms of the genus *Acinetobacter* containing *Acidovorax delafieldii, Acidovax facilis, Acinetobacter* sp., *Acinetobacter calcoaceticus, Acinetobacter lwoffii,* etc., microorganisms of the genus *Aeromonas* containing *Actinomyces* sp., *Aeromonas caviae, Aeromonas hydrophila, Aeromonas salmonicida,* etc., microorganisms of the genus *Alcaligenes* containing *Alcaligenes aestus, Alcaligenes denitrificans, Alcaligenes eutrophus*(after renamed as *Ralstonia eutropha,* it is renamed as *Wautersia eutropha), Alcaligenes faecalis, Alcaligenes latus, Alcaligenes pacificus, Alcaligenes paradoxus, Alcaligenes venestus,* etc. , microorganisms of the genus *Amoebobacter* containing *Alteromonas macleodii, Amoebobacter roseu, Amoebobacter pendens,* etc., microorganisms of the genus *Azospirillum* containing *Aphanocapa* sp., *Aphanothece* sp., *Aquaspirillum autotrophicum, Azorhizobium caulinodans*, *Azospirillum* sp., *Azospirillum brasilense, Azospirillum lipoferum,* etc., microorganisms of the genus *Azotobacter* containing *Azotobacter* sp., *Azotobacter agilis, Azotobacter chroococcum, Azotobacter macrocytogenes, Azotobacter vinelandii*, etc., microorganisms of the genus *Bacillus* containing *Bacillus anthracis, Bacillus cereus, Bacillus megaterium, Bacillus subtillus, Bacillus thuringiensis,* etc., microorganisms of the genus *Beggiatoa* containing *Beggiatoa* sp., *Beggiatoa alba,* etc., microorganisms of the genus *Beijerinckia* containing *Beijerinckia indicus, Beijerinckia mobilis,* etc., microorganisms of the genus *Beneckea* containing *Beneckea natrigens, Beneckea pelagia,* etc., microorganisms of the genus *Caulobacter* containing *Bordetella pertussis, Bradyrhizobium japonicum, Caryophamon latum, Caulobacter bacteroides, Caulobacter crescentus,* etc., microorganisms of the genus *Chlorogloea* containing *Chloroflexus aurantiacus, Chlorogloea fritschii,* etc., microorganisms of the genus *Chromatium* containing *Chromatium minutissimum, Chromatium okenii, Chromatium tepidum,* etc., microorganisms of the genus *Chromobacterium* containing *Chromobacterium violaceum,* etc., microorganisms of the genus *Clostridium* containing *Clostridium botulinum, Clostridium sphenoides,* etc., microorganisms of the genus *Comamonas* containing *Comamonas acidovorans, Comamonas testosteroni,* etc., microorganism of the genus *Corynebacterium* containing *Corynebacterium autotrophicum, Corynebacterium hydrocarboxydans,* etc., microorganisms of the genus *Derxia* containing *Cyanobacteria, Derxia gummosa,* etc., microorganisms of the genus *Desulfonema* containing *Desulfococcus multivorans, Desulfonema limicola, Desulfonema magnum,* etc., microorganisms of the genus *Ectothiorhodospira* containing *Desulfosacina variabilis, Desulfovibrio sapovorans, Ectothiorhodospira halochloris, Ectothiorhodospira mobilis, Ectothiorhodospira vacuolata,* etc., microorganisms of the genus *Halobacterium* containing *Ferrobacillus ferroxidans, Flavobacterium* sp., *Haemophilus influenzae, Halobacterium gibbonsii, Halobacterium volcanii,* etc., microorganisms of the genus *Hydrogenophaga* containing *Haloferax mediterranei, Hydroclathratus clathratus, Hydrogenomonas facilis, Hydrogenophaga flava, Hydrogenophaga pseudoflava, Hydrogenophaga taeniospiralis,* etc., microorganisms of the genus *Hyphomicrobium* containing *Hyphomicrobium vulgare,* etc., microorganisms of the genus *Methylbacterium* containing *Ilyobater delafieldii, Labrys monachus, Lamprocystis reseopersicina, Lampropedia hyaline, Legionella* sp., *Leptothrix discophorus, Methylbacterium* AM1, *Methylbacterium extorquens,* etc., microorganisms of the genus *Methylosinus* containing *Methylococcus thermophilus, Methlocystis parvus, Methylomonas methanica, Methylosinus sporium, Methylosinus trichosporium,* etc., microorganisms of the genus *Micrococcus* containing *Methylovibrio soehngenii, Micrococcus denitrificans, Micrococcus halodenitrificans,* etc., microorganisms of the genus *Mycobacterium* containing *Mycobacterium album, Mycobacterium vacae,* etc., microorganisms of the genus Nitrobacter containing *Nitrobacter agilis, Nitrobacter winogradskyi,* etc., microorganisms of the genus *Nocardia* containing *Nocardia alba, Nocardia asteroides, Nocardia lucida, Nocardia rubra,* etc., microorganisms of the genus *Photobacterium* containing *Paracoccus dentrificans, Oscillatoria limosa, Penicillium cyclopium, Photobacterium mandapamensis, Photobacterium phosphoreum,* etc., microorganisms of the genus *Pseudomonas* containing *Physarum ploycephalum* and *Pseudomonas glathei, Pseudomonas indigofera, Pseudomonas lemonieri, Pseudomonas mallei, Pseudomonas marina, Pseudomonas mixta, Pseudomonas oleovorans, Pseudomonas oxalaticus, Pseudomonas pseudoalcaligenes, Pseudomonas aeruginosa, Pseudomonas alcaligenes, Pseudomonas asplenii, Pseudomonas butanovora, Pseudomonas cepacia, Pseudomonas coronafaciens, Pseudomonas dacunhae, Pseudomonas denitrificans, Pseudomonas diminuta, Pseudomonas echinoides, Pseudomonas fluorescens, Pseudomonas putida, Pseudomonas rubrilineas, Pseudomonas saccharophila, Pseudomonas stutzeri, Pseudomonas syringae, Pseudomonas thermophilus, Pseudomonas viridiflava,* etc., microorganisms of the genus *Ralstonia,* microorganisms of the genus *Rhizobium* containing *Rhizobium hedysarum, Rhizobium lupini, Rhizobium meliloti, Rhizobium phaseoli, Rhizobium trifoli,* etc., microorganisms of the genus *Rhodobacillus,* microorganisms of the genus *Rhodobacter* containing *Rhodobacter capsulatus, Rhodobacter sphaeroides,* etc., microorganisms of the genus *Rhodococcus* containing *Rhodococcus rhodochrous,* etc., microorganisms of the genus *Rhodocyclus* containing *Rhodocyclus gelatinosus, Rhodocyclus tenuis,* etc., microorganisms of the genus *Rhodopseudomonas* containing *Rhodomicrobium vannielii* and *Rhodopseudomonas acidophila, Rhodopseudomonas capsulata,* etc., microorganisms of the genus *Rhodospirillum* containing *Rhodospirillum molischianum, Rhodospirillum rubrum,* etc., microorganisms of the genus *Spirillum* containing *Sphingomonas paucimobilis, Spirillum itersomii, Spirillum serpens,* etc., microorganisms of the genus *Spirulina* containing *Spirulina jenneri, Spirulina maxima, Spirulina subsaksa,* etc., microorganisms of the genus *Staphylococcus* containing *Staphylococcus aureus, Staphylococcus epidermidis, Staphylococcus xylosus,* etc., microorganisms of the genus *Stella* containing *Stella humosa, Stella vacuolata,* etc., microorganisms of the genus *Streptomyces* containing *Streptomyces antibioticus, Streptomyces coelicolor,* etc., microorganisms of the genus *Thiobacillus* containing *Syntrophomonas wolfei, Thermophilic cyanobacteria, Thermus thermophilus, Thiobacillus A2, Thiobacillus acidophilus, Thiobacillus versutus,* etc., microorganisms of the genus *Thiocapsa* containing *Thiocapsa pfennigii,* etc., microorganisms of the genus *Zoogloea* containing *Thiocystis violacea, Vibrio parahaemolyticus, Xanthobacter autotrophicus, Xanthomonas maltophilia, Zoogloea ramigera,* etc.

The present invention, in another aspect, relates to a method for preparing polylactate or hydroxyalkanoate-lactate copolymer[poly(hydroxyalkanoate-co-lactate)], the method comprises: culturing the cells in a medium containing latate or lactate and hydroxyalkanoate as a carbon source; and recovering polylactate or hydroxyalkanoate-lactate copolymer from the cultured cells or culturing the plants; and recovering polylactate or hydroxyalkanoate-lactate copolymer from the cultured plants.

In the present invention, hydroxyalkanoate-lactate copolymer[poly(hydroxyalkanoate-co-lactate)] is preferably selected from the group consisting of poly(3HB-co-lactate), poly(4HB-co-lactate), poly(3HP-co-lactate), poly(3HB-co-4HB-co-lactate) and poly(3HP-co-4HB-co-lactate), but it isn't limited thereto. For example, in hydroxyalkanoate-lactate copolymer[poly(hydroxyalkanoate-co-lactate)] according to the present invention, the hydroxyalkanoate may be one or more selected from the group consisting of 3-hydroxybutyrate, 3-hydroxyvalerate, 4-hydroxybutyrate, (D)-3-hydroxycarboxylic acids of the medium chain length(C6-14), 3-hydroxypropionic acid, 3-hydroxyhexanoic acid, 3-hydroxyheptanoic acid, 3-hydroxyoctanoic acid, 3-hydroxynonanoic acid, 3-hydroxydecanoic acid, 3-hydroxyundecanoic acid, 3-hydroxydodecanoic acid, 3-hydroxytetradecanoic acid, 3-hydroxyhexadecanoic acid, 4-hydroxyvaleric acid, 4-hydroxyhexanoic acid, 4-hydroxyheptanoic acid, 4-hydroxyoctanoic acid, 4-hydroxydecanoic acid, 5-hydroxyvaleric acid, 5-hydroxyhexanoic acid, 6-hydroxydodecanoic acid, 3-hydroxy-4-pentenoic acid, 3-hydroxy-4-trans-hexenoic acid, 3-hydroxy-4-cis-hexenoic acid, 3-hydroxy-5-hexenoic acid, 3-hydroxy-6-trans-octenoic acid, 3-hydroxy-6-cis-octenoic acid, 3-hydroxy-7-octenoic acid, 3-hydroxy-8-nonenoic acid, 3-hydroxy-9-decenoic acid, 3-hydroxy-5-cis-dodecenoic acid, 3-hydroxy-6-cis-dodecenoic acid, 3-hydroxy-5-cis-tetradecenoic acid, 3-hydroxy-7-cis-tetradecenoic acid, 3-hydroxy-5,8-cis-cis-tetradecenoic acid, 3-hydroxy-4-methylvaleric acid, 3-hydroxy-4-methylhexanoic acid, 3-hydroxy-5-methylhexanoic acid, 3-hydroxy-6-methylheptanoic acid, 3-hydroxy-4-methyloctanoic acid, 3-hydroxy-5-methyloctanoic acid, 3-hydroxy-6-methyloctanoic acid, 3-hydroxy-7-methyloctanoic acid, 3-hydroxy-6-methylnonanoic acid, 3-hydroxy-7-methylnonanoic acid, 3-hydroxy-8-methylnonanoic acid, 3-hydroxy-7-methyldecanoic acid, 3-hydroxy-9-methyldecanoic acid, 3-hydroxy-7-methyl-6-octenoic acid, malic acid, 3-hydroxysuccinic acid-methylester, 3-hydroxyadipinic acid-methylester, 3-hydroxysuberic acid-methylester, 3-hydroxyazelaic acid- methylester, 3-hydroxysebacic acid-methylester, 3-hydroxysuberic acid-ethylester, 3-hydroxysebacic acid-ethylester, 3-hydroxypimelic acid-propylester, 3-hydroxysebacic acid-benzylester, 3-hydroxy-8-acetoxyoctanoic acid, 3-hydroxy acetoxynonanoic acid, phenoxy-3-hydroxybutyric acid, phenoxy-3-hydroxyvaleric acid, phenoxy-3-hydroxyheptanoic acid, phenoxy-3-hydroxyoctanoic acid, para-cyanophenoxy-3-hydroxybutyric acid, para-cyanophenoxy-3-hydroxyvaleric acid, para-cyanophenoxy-3-hydroxyhexanoic acid, para-nitrophenoxy-3-hydroxyhexanoic acid, 3-hydroxy-5-phenylvaleric acid, 3-hydroxy-5-cyclohexylbutyric acid, 3,12-dihydroxydodecanoic acid, 3,8-dihydroxy-5-cis-tetradecenoic acid, 3-hydroxy-4,5-epoxydecanoic acid, 3-hydroxy-6,7-epoxydodecanoic acid, 3-hydroxy-8,9-epoxy-5,6-cis-tetradecanoic acid, 7-cyano-3-hydroxyheptanoic acid, 9-cyano-3-hydroxynonanoic acid, 3-hydroxy-7-fluoroheptanoic acid, 3-hydroxy-9-fluorononanoic acid, 3-hydroxy-6-chlorohexanoic acid, 3-hydroxy-8-chlorooctanoic acid, 3-hydroxy-6-bromohexanoic acid, 3-hydroxy-8-bromooctanoic acid, 3-hydroxy-11-bromoundecanoic acid, 3-hydroxy-2-butenoic acid, 6-hydroxy-3-dodecenoic acid, 3-hydroxy-2-methylbutyric acid, 3-hydroxy-2-methylvaleric acid, and 3-hydroxy-2,6-dimethyl-5-heptenoic acid.

The present invention, in still another aspect, relates to a recombinant vector for preparing polylactate or hydroxyalkanoate-lactate copolymer[poly(hydroxyalkanoate-co-lactate)] which contains a gene encoding an enzyme converting lactate into lactyl-CoA and a gene encoding PHA synthase capable of using lactyl-CoA as a substrate, and cells or plants transformed with the recombinant vector.

The term "vector" means a DNA construct comprising DNA sequence operably linked to a suitable control sequence capable of expressing the DNA in a suitable host. In the present invention, plasmid vector, bacteriophage vector, cosmid vector, YAC(Yeast Artificial Chromosome) vector can be used as the above vector and it is preferable to use plasmid vector for the purpose of the present invention. Typical plasmid vectors that can be used for the purpose has (a) an origin of replication so that it leads to effective replication in order to have each host cell containing several hundreds of plasmid vector (b) an antibiotic-resistant gene so that a host cell transformed with a plasmid vector can be selected and (c) a structure containing a restriction enzyme cutting site where a foreign DNA fragment is to be inserted. Even though there isn't a suitable restriction enzyme cutting site, a vector and a foreign DNA can be easily ligated by using a synthetic oligonucleotide adaptor or a linker according to the conventional method.

After ligation, a vector must be transformed into a suitable host cell. Prokaryotic cell or eukaryotic cell can be a preferable host cell for the present invention, more preferably, prokaryotic cell. Microorganisms without having aforementioned PHA synthase like *E*. *coli* as well as microorganisms having aforementioned PHA synthase can be used as a suitable prokaryotic cell. Also, *E*. *coli* transformed into a gene encoding PHA synthase can be used as a microorganism having an aforementioned gene encoding PHA synthase. Preferable *E*. *colis* include *E*. *coli* DH5a, *E*. *coli* JM101, *E*. *coli* K12, *E. coli* W3110, *E*. *coli* X1776, *E*. *coli* XL1-Blue(Stratagene) and *E*. *coli* B. But, *E. colis,* such as FMB101, NM522, NM538 and NM539, and other prokaryotic species and genera can also be used. In addition to aforementioned *E*. *coli* and microorganisms having a gene encoding PHA synthase, the genus *Agrobacterium,* such as *Agrobacterium* A4, *Bacilli,* such as *Bacillus subtilis,* enterobacteria, such as *Salmonellla typhimurium* or *Serratia marcescens* can be used as a host cell. The universal eukaryotic host cells, such as yeast, fungi, insect cells, such as *spodoptera frugiperda* (SF9), animal cells, such as CHO and mouse cell, tissue cultured human cells and plant cells can also be used. If transformed into a suitable host, a vector can replicate and function regardless of a host genome, or can be incorporated into a genome itself in several cases.
As is generally known in the art, to have a high expression level of a transformed gene in a host cell, the gene must be operably linked to a transcription, translation and expression regulating sequences functioning in a selected expression host. The expression control sequence and the corresponding gene are preferably contained in one expression vector containing both a bacterial selection marker and a replication origin. In the case where an expression host cell is eukaryotic cell, the expression vector must additionally contain an expression marker useful in a eukaryotic expression host.

The transformed cell constitutes another aspect of the present invention by the aforementioned expression vector. In the present invention, the term "transformation" means that DNA can be replicated as a factor outside of chromosome or by means of completion of the entire chromosome by introducing DNA as a host.

It is to be understood that not all vectors and expression regulating sequences function equally in expressing DNA sequences of the present invention. Also, not all hosts function equally for an equal expression system. However, a person skilled in the art can suitably select from several vectors, expression control sequences and hosts without excessive experimental burden within the scope of the present invention. For example, a host must be considered in selecting a vector because the vector must be replicated in the host. The replication number of a vector, the ability of regulating replication number and the expression of other protein encoded by the corresponding vector(for example, the expression of an antibiotic marker) should also be considered. In the range of these variables, a person skilled in the art can select various vector/expression regulating sequence/host combinations which are suitable for the present invention.

Transformation of plants can be achieved by the conventional method using *Agrobacterium* or virus vectors. For example, transformed plants can be obtained by transforming the microorganism of the genus *Agrobacterium* with a recombinant vector containing the inventive gene and infecting a tissue, etc. of the target plants with the transformed microorganism of the genus *Agrobacterium.* For example, transformed plants suitable for the present invention can be obtained by a method that is the same or similar to the preceding patent (WO 94/11519; US 6, 103, 956) relating to PHA preparation using transformed plants.

Transformed plants that could be used in the present invention include tobacco, tomato, red peppers, beans, rice, corn, but it isn't limited thereto. Also, even though the transformed plants are sexually propagated plants, it is obvious to a person skilled in the art that they can be asexually reproduced by tissue culture, etc.

The present invention, in yet another aspect, relates to a method for preparing polylactate or hydroalkanoate-lactate copolymer[poly(hydroalkanoate-co-lactate)], the method comprising the steps of culturing the transformed cells in a medium containing lactate or hydroalkanoate and lactate as a carbon source; and recovering polylactate or hydroxyalkanoate-lactate copolymer from the cultured cell or culturing the transformed plants; and recovering polylactate or hydroxyalkanoate-lactate copolymer from the cultured plants.

The present invention, in yet another aspect, relates to hydroalkanoate-lactate copolymer[poly(hydroalkanoate-co-lactate)] containing, as a monomer, lactate and one or more hydroalkanoate selected from the group consisting of 3-hydroxybutyrate, 3-hydroxyvalerate, 4-hydroxybutyrate, (D)-3-hydroxycarboxylic acids of the medium chain length(C6-14), 3-hydroxypropionic acid, 3-hydroxyhexanoic acid, 3-hydroxyheptanoic acid, 3-hydroxyoctanoic acid, 3-hydroxynonanoic acid, 3-hydroxydecanoic acid, 3-hydroxyundecanoic acid, 3-hydroxydodecanoic acid, 3-hydroxytetradecanoic acid, 3-hydroxyhexadecanoic acid, 4-hydroxyvaleric acid, 4-hydroxyhexanoic acid, 4-hydroxyheptanoic acid, 4-hydroxyoctanoic acid, 4-hydroxydecanoic acid, 5-hydroxyvaleric acid, 5-hydroxyhexanoic acid, 6-hydroxydodecanoic acid, 3-hydroxy-4-pentenoic acid, 3-hydroxy-4-trans-hexenoic acid, 3-hydroxy-4-cis-hexenoic acid, 3-hydroxy-5-hexenoic acid, 3-hydroxy-6-trans-octenoic acid, 3-hydroxy-6-cis-octenoic acid, 3-hydroxy-7-octenoic acid, 3-hydroxy-8-nonenoic acid, 3-hydroxy-9-decenoic acid, 3-hydroxy-5-cis-dodecenoic acid, 3-hydroxy-6-cis-dodecenoic acid, 3-hydroxy-5-cis-tetradecenoic acid, 3-hydroxy-7-cis-tetradecenoic acid, 3-hydroxy-5,8-cis-cis-tetradecenoic acid, 3-hydroxy-4-methylvaleric acid, 3-hydroxy-4-methylhexanoic acid, 3-hydroxy-5-methylhexanoic acid, 3-hydroxy-6-methylheptanoic acid, 3-hydroxy-4-methyloctanoic acid, 3-hydroxy-5-methyloctanoic acid, 3-hydroxy-6-methyloctanoic acid, 3-hydroxy-7-methyloctanoic acid, 3-hydroxy-6-methylnonanoic acid, 3-hydroxy-7-methylnonanoic acid, 3-hydroxy-8-methylnonanoic acid, 3-hydroxy-7-methyldecanoic acid, 3-hydroxy-9-methyldecanoic acid, 3-hydroxy-7-methyl-6-octenoic acid, malic acid, 3-hydroxysuccinic acid-methylester, 3-hydroxyadipinic acid- methylester, 3-hydroxysuberic acid- methylester, 3-hydroxyazelaic acid- methylester, 3-hydroxysebacic acid- methylester, 3-hydroxysuberic acid-ethylester, 3-hydroxysebacic acid-ethylester, 3-hydroxypimelic acid-propylester, 3-hydroxysebacic acid-benzylester, 3-hydroxy-8-acetoxyoctanoic acid, 3-hydroxy-9-acetoxynonanoic acid, phenoxy-3-hydroxybutyric acid, phenoxy-3-hydroxyvaleric acid, phenoxy-3-hydroxyheptanoic acid, phenoxy-3-hydroxyoctanoic acid, para-cyanophenoxy-3-hydroxybutyric acid, para-cyanophenoxy-3-hydroxyvaleric acid, para-cyanophenoxy-3-hydroxyhexanoic acid, para-nitrophenoxy-3-hydroxyhexanoic acid, 3-hydroxy-5-phenylvaleric acid, 3-hydroxy-5-cyclohexylbutyric acid, 3,12-dihydroxydodecanoic acid, 3,8-dihydroxy-5-cis-tetradecenoic acid, 3-hydroxy-4,5-epoxydecanoic acid, 3-hydroxy-6,7-epoxydodecanoic acid, 3-hydroxy-8,9-epoxy-5,6-cis-tetradecanoic acid, 7-cyano-3-hydroxyheptanoic acid, 9-cyano-3-hydroxynonanoic acid, 3-hydroxy-7-fluoroheptanoic acid, 3-hydroxy-9-fluorononanoic acid, 3-hydroxy-6-chlorohexanoic acid, 3-hydroxy-8-chlorooctanoic acid, 3-hydroxy-6-bromohexanoic acid, 3-hydroxy-8-bromooctanoic acid, 3-hydroxy-11-bromoundecanoic acid, 3-hydroxy-2-butenoic acid, 6-hydroxy-3-dodecenoic acid, 3-hydroxy-2-methylbutyric acid, 3-hydroxy-2-methylvaleric acid and 3-hydroxy-2,6-dimethyl-5-heptenoic acid.

In the present invention, the hydroalkanoate preferably contains 3HB and 4HB or 3HP and 4HB.

The present invention, in yet another aspect, provides a copolymer[poly(3HA-co-lactate)] of 3HA having three to twelve carbons and lactate.

In the present invention, the copolymer of 3HA having three to twelve carbons and lactate is preferably [poly(3HHx-co-3HO-co-3HD-co-lactate)] or [poly(3HHx-co-3HO-co-3HD-co-3HDD-lactate)].

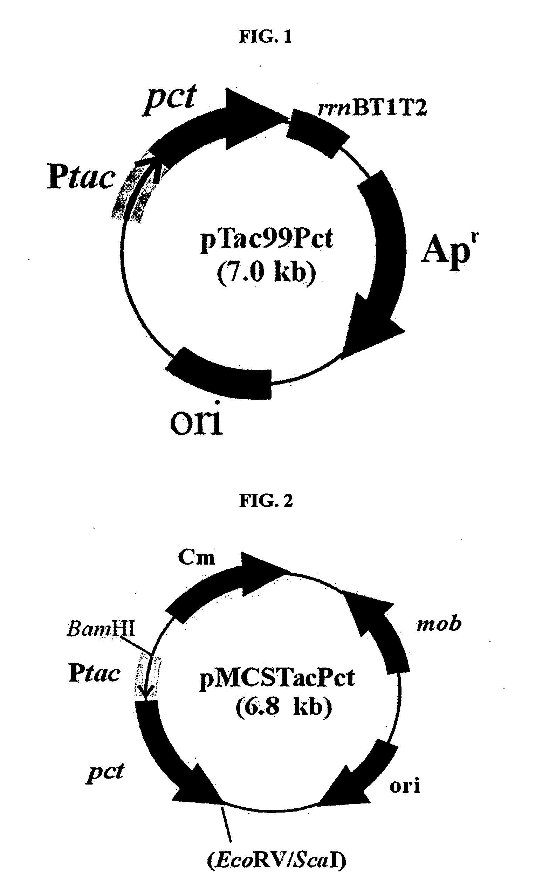

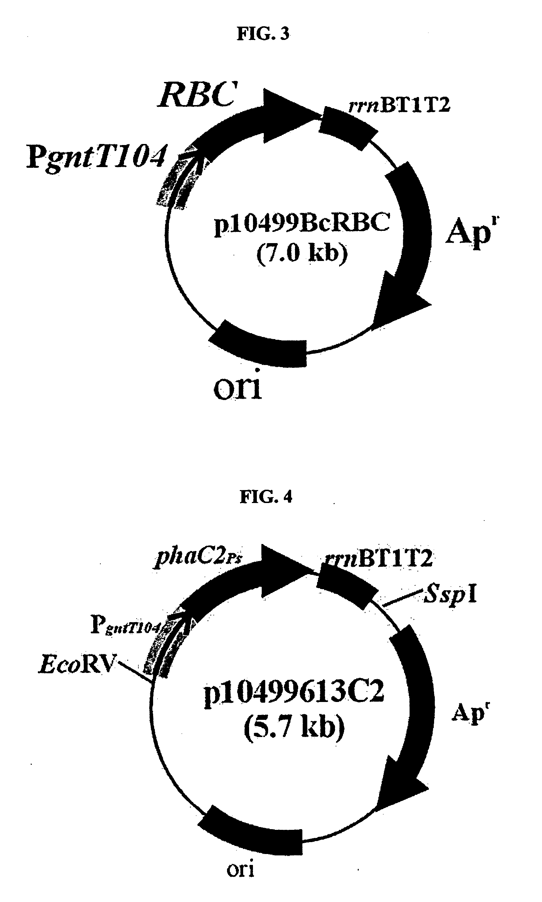

In the present invention, after a gene encoding an enzyme converting lactate into lactyl-CoA(*pct*) was inserted into pTac99A to construct an expression vector pTac99Pct (FIG. 1), the gene fragment containing tac promoter, the pct gene, transcription terminator from the vector pTac99Pct was inserted into pBBRIMCS to construct a recombinant expression vector pMCSTacPct (FIG. 2). Also, a gene encoding PHA synthase drived from *Bacillus cereus* ATCC 14579(*phaRBC*) was inserted into p10499A vector to construct a recombinant expression vector p10499BcRBC (FIG. 3). In addition, a gene encoding PHA synthase derived from *Pseudomonas* SP. 61-3*(phaC)* was amplified and inserted into p10499A to construct a recombinant expression vector p10499613C2 (FIG. 4).

Meanwhile, it is well known that various PHA and its copolymers can be prepared according to the kinds of carbon sources used (KR 10-250830 B1; Lee, Biotechnol. Bioeng., 49:1, 1996; Steirlbchel and Valentin, FEMS Microbiol. Lett., 128:219, 1995). All hydroxyalkanoates including (3-hydroxypropionate(3HP), 4-hydroxybutyrate(4HB), 3-hydroxybutyrate(3HB), 3-hydroxyhexanoate (3HHx), 3-hydroxyoctanoate(3HO), 3-hydroxydecanoate(3HD), 3-hydroxydodecanoate(3HDD), etc.) can comprise as a PHA monomer. It is possible to prepare PHA comprised of various monomer and molecular composition ratio. That is, according to a carbon source used in culture, hydroxyalkanoate-lactate-copolymer which contains hydroxyalkanoate, such as 3-hydroxybutyrate, 3-hydroxyvalerate, 4-hydroxybutyrate, (D)-3-hydroxycarboxylic acids of the medium chain length(C6-14), 3-hydroxypropionic acid, 3-hydroxyhexanoic acid, 3-hydroxyheptanoic acid, 3-hydroxyoctanoic acid, 3-hydroxynonanoic acid, 3-hydroxydecanoic acid, 3-hydroxyundecanoic acid, 3-hydroxydodecanoic acid, 3-hydroxytetradecanoic acid, 3-hydroxyhexadecanoic acid, 4-hydroxyvaleric acid, 4-hydroxyhexanoic acid, 4-hydroxyheptanoic acid, 4-hydroxyoctanoic acid, 4-hydroxydecanoic acid, 5-hydroxyvaleric acid, 5-hydroxyhexanoic acid, 6-hydroxydodecanoic acid, 3-hydroxy-4-pentenoic acid, 3-hydroxy-4-trans-hexenoic acid, 3-hydroxy-4-cis-hexenoic acid, 3-hydroxy-5-hexenoic acid, 3-hydroxy-6-trans-octenoic acid, 3-hydroxy-6-cis-octenoic acid, 3-hydroxy-7-octenoic acid, 3-hydroxy-8-nonenoic acid, 3-hydroxy-9-decenoic acid, 3-hydroxy-5-cis-dodecenoic acid, 3-hydroxy-6-cis-dodecenoic acid, 3-hydroxy-5-cis-tetradecenoic acid, 3-hydroxy-7-cis-tetradecenoic acid, 3-hydroxy-5,8-cis-cis-tetradecenoic acid, 3-hydroxy-4-methylvaleric acid, 3-hydroxy-4-methylhexanoic acid, 3-hydroxy-5-methylhexanoic acid, 3-hydroxy-6-methylheptanoic acid, 3-hydroxy-4-methyloctanoic acid, 3-hydroxy-5-methyloctanoic acid, 3-hydroxy-6-methyloctanoic acid, 3-hydroxy-7-methyloctanoic acid, 3-hydroxy-6-methylnonanoic acid, 3-hydroxy-7-methylnonanoic acid, 3-hydroxy-8-methylnonanoic acid, 3-hydroxy-7-methyldecanoic acid, 3-hydroxy-9-methyldecanoic acid, 3-hydroxy-7-methyl-6-octenoic acid, malic acid, 3-hydroxysuccinic acid-methylester, 3-hydroxyadipinic acid- methylester, 3-hydroxysuberic acid- methylester, 3-hydroxyazelaic acid- methylester, 3-hydroxysebacic acid- methylester, 3-hydroxysuberic acid-ethylester, 3-hydroxysebacic acid- ethylester, 3-hydroxypimelic acid-propylester, 3-hydroxysebacic acid-benzylester, 3-hydroxy-8-acetoxyoctanoic acid, 3-hydroxy-9-acetoxynonanoic acid, phenoxy-3-hydroxybutyric acid, phenoxy-3-hydroxyvaleric acid, phenoxy-3-hydroxyheptanoic acid, phenoxy-3-hydroxyoctanoic acid, para-cyanophenoxy-3-hydroxybutyric acid, para-cyanophenoxy-3-hydroxyvaleric acid, para-cyanophenoxy-3-hydroxyhexanoic acid, para-nitrophenoxy-3-hydroxyhexanoic acid, 3-hydroxy-5-penylvaleric acid, 3-hydroxy-5-cyclohexylbutyric acid, 3,12-dihydroxydodecanoic acid, 3,8-dihydroxy-5-cis-tetradecenoic acid, 3-hydroxy-4,5-epoxydecanoic acid, 3-hydroxy-6,7-epoxydodecanoic acid, 3-hydroxy-8,9-epoxy-5,6-cis-tetradecanoic acid, 7-cyano-3-hydroxyheptanoic acid, 9-cyano-3-hydroxynonanoic acid, 3-hydroxy-7-fluoroheptanoic acid, 3-hydroxy-9-fluorononanoic acid, 3-hydroxy-6-chlorohexanoic acid, 3-hydroxy-8-chlorooctanoic acid, 3-hydroxy-6-bromohexanoic acid, 3-hydroxy-8-bromooctanoic acid, 3-hydroxy-11-bromoundecanoic acid, 3-hydroxy-2-butenoic acid, 6-hydroxy-3-dodecenoic acid, 3-hydroxy-2-methylbutyric acid, 3-hydroxy-2-methylvaleric acid and 3-hydroxy-2,6-dimethyl-5-heptenoic acid, can be prepared. Also, copolymers, which comprise hydroxyalkanoate having double bonds or triple bonds as a monomer in addition to the above single bond compound, can be prepared. Therefore, "hydroxyalkanoate" used in the present invention is defined to include hydroxyalkanoate which has a double bond or a triple bond in addition to the above single bond compound.

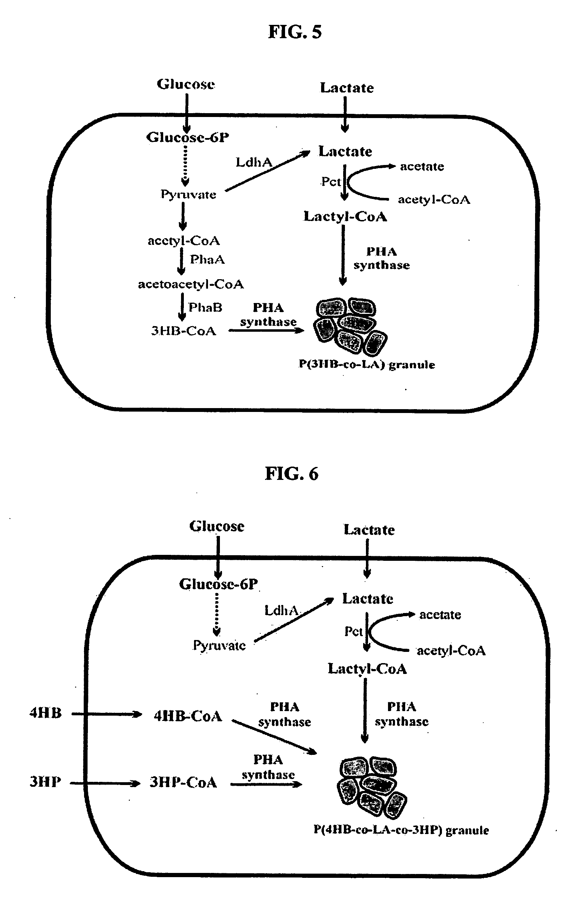

Therefore, in the case where cells or plants transformed with the recombinant vectors of the present invention are used, it is possible to prepare copolymers [poly(hydroxyalkanoate-co-lactate)] of lactate and various hydroxyalkanoates as well as PLA (FIG. 5 - FIG. 8).
In the case where *Ralstonia eutropha* NCIMB 11599 containing a gene encoding PHA synthease using lactyl-CoA as a substrate is transformed with a plasmid containing propionyl-CoA transferase(*pct*) to obtain a recombinant *Ralstonia eutropha* and the recombinant *Ralstonia eutropha* is cultured in a medium containing lactate or lactate and various carbon sources, various combinations of copolymers can be prepared. For example, when the above recombinant *Ralstonia eutropha* is cultured in a production medium containing lactate, poly(3HB-co-lactate) can be prepared (FIG. 5). Also, when the above recombinant *Ralstonia eutropha* is cultured in a production medium containing 3HB, 4HB and lactate, poly(3HB-co-4HB-co-lactate) can be prepared (FIG. 6).

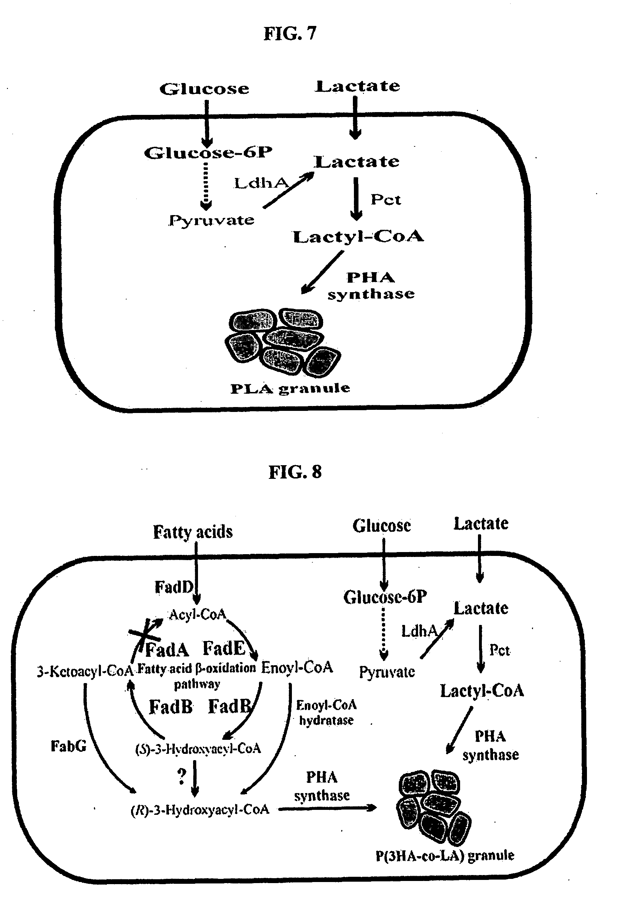

In the case where a recombinant *E*. *coli* transformed with a recombinant vector *containing phaRBC* derived from *Bacillus cereus(ATCC* 14579) and a recombinant vector containing *pct* (or a recombinant *E*. *coli* transformed with a recombinant vector containing both *phaRBC* and *pct)* is cultured in a production medium supplemented with lactate, polylactate can be prepared (FIG. 7). Also, in the case where the above recombinant *E*. *coli* is cultured in a medium supplemented with lactate and various carbon sources, various combinations of copolymers can be prepared.

Meanwhile, it was confirmed in KR 10-0447531B1 that in the case where a recombinant *E*. *coli* WB101 transformed with p10499613C2 was cultured in media containing decenoic acid and dodecenoic acid, respectively, a copolymer containing 3HHx, 3HO and 3HD and a copolymer containing 3HHx, 3HO, 3HD and 3HDD were prepared. Therefore, in the case where a recombinant *E*. *coli* transformed with a recombinant vector containing a gene encoding PHA synthase(*phaC*) derived from *Pseudomonas* sp. 61-3 and a recombinant vector containing *pct(or* a recombinant *E*. *coli* transformed with a recombinant vector containing both *phaC* and *pct)* are cultured in a production medium containing lactate and fatty acid, a copolymer[poly(3HA-co-lactate)] containing MCL-PHA monomers(C3-12) and lactate can be prepared (FIG. 8). For example, in the case where the recombinant *E*. *coli* is cultured in media containing lactate and decenoic acid, respectively, [poly(3HHx-co-3HO-co-3HD-co-lactate)] can be prepared, and in the case where the recombinant *E*. *coli* is cultured in media containing lactate and dodecenoic acid, respectively, [poly(3HHx-co-3HO-co-3HD-co-3HDD-lactate)] can be prepared.

The media used for preparing biologic polylactate and its copolymer isn't specifically limited as far as it doesn't obstruct the purpose of the present invention. In the present invention, a complex medium, Luria-Bertani(LB) medium was used when recombinant *E*. *coli* is cultured and a basic medium, N-limited MR medium was used when recombinant *Ralstonia eutropha* is cultured.

### Examples

Hereinafter, the present invention will be described in more detail by specific examples. However, the present invention is not limited to these examples, and it is obvious to those skilled in the field of the present invention that numerous variations or modifications could be made within the spirit and scope of the present invention.

Although *pct* was used as a gene encoding an enzyme converting lactate into lactyl-CoA to provide PHA polymerase substrate, lactyl-CoA in below examples, it is obvious to those skilled in the art of the present invention that transferase derived from other microorganisms can result in the same result.

Also, although the below examples exemplified *phaC* derived from *Pseudomonas* sp. 61-3 and *phaRBC* derived from *Bacillus cereus(ATCC* 14579) as a gene encoding PHA synthase capable of using lactyl-CoA as a substrate, it is obvious to those skilled in the art of the present invention that the same result can be obtained when PHA synthase having a similar substrate specificity, derived from various kinds of microorganisms, such as *Wautersia eutropha, Alcaligenes latus, Sinorhizobium meliloti, Bacillus megaterium, Chromatium vinosum,* etc. is used.

Also, although the below examples exemplified *E. coli* and *Ralstonia eutropha* as microorganisms without having a gene encoding PHA synthase and microorganisms having a gene encoding PHA synthase, respectively, it is obvious to those skilled in the art of the present invention that the same result can be obtained when other kinds of cells(bacteria, yeast, fungi, animal cells, plant cells) and plants are used.

### Example 1: Construction of recombinant vector containing pct

Primers having basic sequences of SEQ ID NOs: 1 and 2 were synthesized based on *pct* gene sequence (Selmer et al., Eur J. Biochem., 269:372,2002) for PCR cloning of *pct:*
SEQ ID NO: 1: 5-ggaattcATGAGAAAGGTTCCCATTATTACCGCAGATGA
SEQ ID NO: 2: 5-gctctagattaggacttcatttccttcagacccattaagccttctg

To amplify *pct,* PCR was carried out using chromosome DNA of *Clostridium propionicum* as template and the above primers of SEQ ID NOs: 1 and 2. PCR was performed under the condition of 30 cycles(denaturation at 94 °C, for 50 sec annealing at 52 °C, for 50 sec extension at 72 °C, for 2 min). As a result of examing PCR reaction product by agarose gelelectrophoresis, 1.5 Kb gene fragment corresponding to *pct* was observed. After pTac99A vector (Park and Lee, J. Bacteriol., 185:5391, 2003) was digested with *Eco*RI/*Xba*I enzyme, pTac99Pct recombinant expression vector was prepared by inserting *pct* into *Eco*RI*lXba*I recognition site of pTac99A (FIG. 1). Also, pMCSTacPct was prepared by inserting a gene fragment obtained by digesting pTac99Pct with *Bam*HI*lSca*I into pBRRIMCS (Kovach et al., Gene, 166:175, 1995) digested with Bam*HI*/EcoR*V* (FIG 2).

### Example 2: Construction of recombinant vector containing a gene encoding PHA synthase

To amplify *phaRBC* operon, PCR was carried out using chromosome DNA of *Bacillus cereusas* as template and primers of SEQ ID NOs: 3 and 4. PCR was performed under the condition of 30 cycles(denaturation at 94 °C, for 50 sec annealing at 52 °C, for 50 sec extension at 72 °C, for 2 min),
SEQ ID NO: 3: 5-ggaattcatgaattgtttcaaaaacga
SEQ ID NO: 4: 5-cgggatccttaattagaacgctcttcaa

As a result of examining PCR reaction product by agarose gelelectrophoresis, 2.5 Kb gene fragment corresponding to *phaRBC* was observed. After p10499A vector(KR 10-0447531B1) was digested with *Eco*RI/*Bam*HI enzyme, p10499BcRBC recombinant expression vector was prepared by inserting *phaRBC* into *Eco*RI/ *Bam*HI recognition site of p10499A (FIG. 3).

Also, a gene encoding PHA polymerase(*phaC*) derived from *Pseudomonas* sp. 61-3 was amplified and inserted into p10499A to prepare a recombinant expression vector p10499613C2(KR 10-0447531B1) (FIG. 4).

### Example 3: Construction of recombinant Ralstonia eutropha and preparation of hydroalkanoate-lactate copolymer using the same

After recombinant *Ralstonia eutropha(pMCSTacPct)* was constructed by introducing pMCSTacPct prepared in Example 1 into *Ralstonia eutropha* NCIMB11599, two-step culture was carried out. After recombinant *Ralstonia eutropha* was cultured in a NB medium(Nutrient Broth; 5g/L of Peptone, 3g/L of Beef extract) overnight, the strains recovered by centrifugation were cultured in a N-limited MR medium containing 2g/L of lactate for 3 days. The composition of N-limited MR medium was as follows: 6.67g of KH₂PO₄, 4g of (NH₄)₂HPI₄, 0.8g of MgSO₄ • 7H₂O, 0.8g of citric acid, and 5mL of trace metal solution. Trace metal solution(per liter): 5mL of 5M HCl, 10g of FeSO₄ • 7H₂O, 2g of CaCl₂, 2.2g of ZnSO₄ • 7H₂O, 0.5g of MnSO₄ • 4H₂O, 1g of CuSO₄ • 5H₂O, 0.1g of (NH₄)₆Mo₇O₂ • 4H₂O, and 0.02g of Na₂B₄O₂ • 10H₂O.

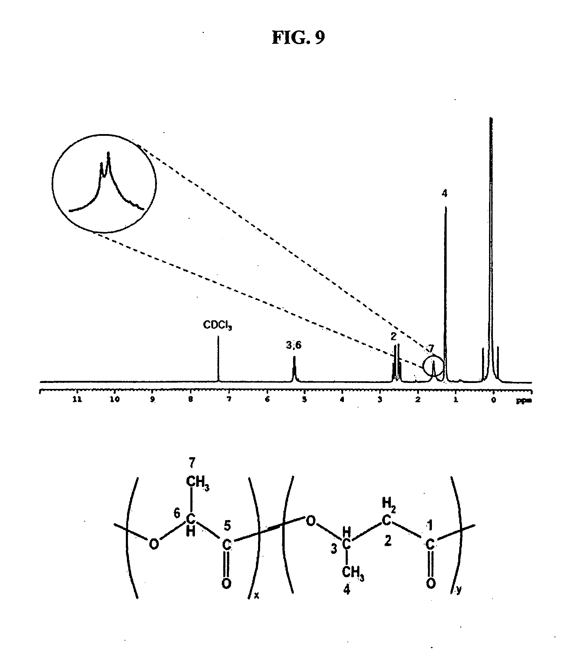

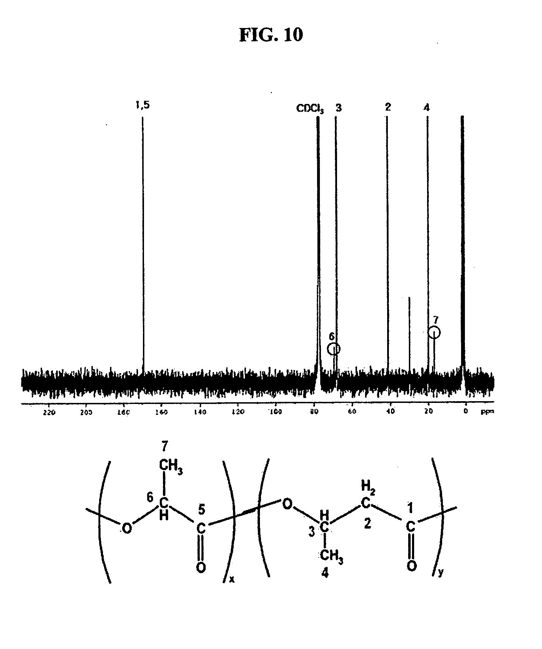

After the strains were recovered by centrifugation, and freeze-dried, polymer substance accumulated in the strains was recovered using chloroform. As a result of NMR analysis on recovered polymer, it was confirmed that the above recovered polymer substance was poly(3HB-co-lactate) copolymer as shown in FIG. 9, FIG. 10.

### Example 4: Construction of recombinant E. coli and preparation of copolymer of polylactate and hydroxyalkanoate-lactate using_the same

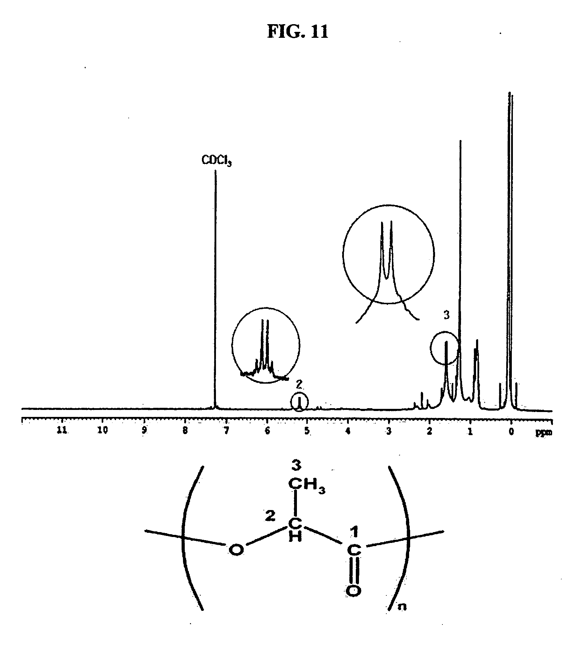

Recombinant *E*. *coli* was constructed by introducing pMCSTacPct prepared in Example 1 and p10499BcRBC prepared in Example 2 into *E*. *coli* XL1-Blue and cultured in an LB medium containing 2g/L of lactate for 4 days, followed by centrifuging to recover the strains. The recovered strains were freeze-dried to recover polymer substance accumulated in the strains using chloroform. As a result of NMR analysis on the recovered polymer, it was confirmed that the above obtained polymer substance was polylactate (FIG. 11).

Also, a recombinant *E*. *coli* was constructed by introducing pMCSTacPct prepared in Example 1 and p10499613C2 produced in Example 2 into *E*. *coli* WB101 [W3110(fadB::Km). WB101 is a strain that is identified to be effective in producing MCL-PHA as fadB mutant *E*. *coli(*KR 10-044753B1). The recombinant *E. coli* WB101 was cultured in LB media containing 2g/L of lactate and 2 g/L of decenoic acid for 4 days, respectively and centrifuged to recover strains. The recovered strains were freeze-dried to recover polymer substance accumulated in the strains using chloroform. As a result of NMR analysis on the obtained polymer substance, it was confirmed that the above recovered polymer was [poly(3HHx-co-3HO-co-3HD-co-lactate)] copolymer.

### INDUSTRIAL APPLICABILITY

As described above in detail, the present invention has an effect of providing cells or plants which express a gene encoding an enzyme converting lactate into lactyl-CoA and a gene encoding PHA synthase capable of using lactyl-CoA as a substrate, and a method for preparing polylactate or hydroxyalkanoate-lactate copolymer using the same. According to the present invention, polylactate can be prepared by using cells or plants and various kinds of polyesters containing lactate and various hydroxyalkanoates as a monomer can be also prepared.
## Claims

1. Transformed cells having the ability of producing polylactate or hydroxyalkanoate-lactate copolymer [poly(hydroxyalkanoate-co-lactate)], which contains a gene encoding propionyl-CoA transferase and a gene encoding polyhydroxyalkanoates synthase selected from phaC and phaRBC,
wherein said transformed cells are obtained by transforming cells without having a gene encoding polyhydroxyalkanoates synthase selected from phaC and phaRBC with a recombinant vector containing a gene encoding propionyl-CoA transferase and a gene encoding polyhydroxyalkanoates synthase selected from phaC and phaRBC.

2. Transformed cells having the ability of producing polylactate or hydroxyalkanoate-lactate copolymer [poly(hydroxyalkanoate-co-lactate)], which contains a gene encoding propionyl-CoA transferase and a gene encoding polyhydroxyalkanoates synthase selected from phaC and phaRBC,
wherein said transformed cells are obtained :
> by transforming cells having a gene encoding polyhydroxyalkanoates synthase selected from phaC and phaRBC with a recombinant vector containing a gene encoding propionyl-CoA transferase; or
> by transforming cells having a gene encoding propionyl-CoA transferase with a recombinant vector containing a gene encoding polyhydroxyalkanoates synthase selected from phaC and phaRBC.

3. The transformed cells according to claim 1, wherein said cells are transformed with a recombinant vector containing propionyl-CoA transferase, at the same time, transformed with a vector containing phaC or phaRBC, or phaC or phaRBC are inserted onto chromosome thereof.

4. The transformed cells according to claim 1, wherein the cells without having a gene encoding polyhydroxyalkanoates synthase is *E*. *coli.*

5. The transformed cells according to claim 2, wherein said cells having a gene encoding propionyl-CoA transferase are amplified with said gene.

6. The transformed cells according to claim 2, wherein said cells having a gene encoding propionyl-CoA transferase are *Clostridium propionicum.*

7. A method for preparing polylactate or copolymer of poly(3HB-co-lactate), the method comprises: culturing the cells of any one of claims 1 to 6 in a medium containing lactate, or lactate and various kinds of hydroxyalkanoates as a carbon source; and recovering polylactate or copolymer of poly(3HB-co-lactate) from the cultured cells.

8. A method for preparing a copolymer[poly(3HHx-co-3HO-co-3HD-co-lactate)] and lactate, the method comprises: culturing the cells of claim 1 in a medium containing lactate and fatty acid as a carbon source; and recovering the copolymer[poly(3HHx-co-3HO-co-3HD-co-lactate)] and lactate from the cultured cells, wherein said fatty acid is decenoic acid.

9. A recombinant vector containing a gene encoding propionyl-CoA transferase gene and a gene encoding polyhydroxyalkanoates synthase selected from phaC and phaRBC.

10. The recombinant vector according to claim 9, wherein phaC is derived from *Pseudomonas sp.*

11. The cells transformed with the recombinant vector of any one of claims 9 to 10.

12. A method for preparing polylactate or copolymer of poly(3HB-co-lactate), the method comprises: culturing the transformed cells of claim 11 in a medium containing lactate or lactate and various kinds of hydroxyalkanoates as a carbon source; and recovering polylactate or copolymer of poly(3HB-co-lactate) from the cultured cells.

## Patentansprüche

1. Transformierte Zellen, welche die Fähigkeit haben, Polylactat oder Hydroxyalkanoat-Lactat-Copolymer [Poly(hydroxyalkanoat-Co-Lactat)] zu produzieren, welche ein Propionyl-CoA-Transferase codierendes Gen und ein Polyhydroxyalkanoatsynthase codierendes Gen ausgewählt aus phaC und phaRBC enthalten, wobei die transformierten Zellen erhalten werden durch Transformieren von Zellen, welche kein Polyhydroxyalkanoatsynthase codierendes Gen ausgewählt aus phaC und phaRBC haben, mit einem rekombinanten Vektor, welcher ein Propionyl-CoA-Transferase codierendes Gen und ein Polyhydroxyalkanoatsynthase codierendes Gen ausgewählt aus phaC und phaRBC enthält.

2. Transformierte Zellen, welche die Fähigkeit haben, Polylactat oder Hydroxyalkanoat-Lactat-Copolymer [Poly(hydroxyalkanoat-Co-Lactat)] zu produzieren, welche ein Propionyl-CoA-Transferase codierendes Gen und ein Polyhydroxyalkanoatsynthase codierendes Gen ausgewählt aus phaC und phaRBC enthalten,
wobei die transformierten Zellen erhalten werden:
- durch Transformieren von Zellen, welche ein Polyhydroxyalkanoatsynthase codierendes Gen ausgewählt aus phaC und phaRBC haben, mit einem rekombinanten Vektor, welcher ein Propionyl-CoA-Transferase codierendes Gen enthält; oder
- durch Transformieren von Zellen, welche ein Propionyl-CoA-Transferase codierendes Gen haben, mit einem rekombinanten Vektor, welcher ein Polyhydroxyalkanoatsynthase codierendes Gen ausgewählt aus phaC und phaRBC enthält.

3. Transformierte Zellen nach Anspruch 1, wobei die Zellen mit einem rekombinanten Vektor, welcher Propionyl-CoA-Transferase enthält, transformiert sind, und zur gleichen Zeit mit einem Vektor, welcher phaC oder phaRBC enthält, transformiert sind, oder phaC oder phaRBC auf dem Chromosom davon inseriert sind.

4. Transformierte Zellen nach Anspruch 1, wobei die Zellen, die kein Polyhydroxyalkanoatsynthase codierendes Gen haben, *E. coli sind.*

5. Transformierte Zellen nach Anspruch 2, wobei die Zellen, welche ein Propionyl-CoA-Transferase codierendes Gen haben, mit dem Gen amplifiziert sind.

6. Transformierte Zellen nach Anspruch 2, wobei die Zellen, welche ein Propionyl-CoA-Transferase codierendes Gen haben, *Clostridium propionicum* sind.

7. Verfahren zur Herstellung von Polylactat oder einem Copolymer von Poly(3HB-Co-lactat), wobei das Verfahren umfasst: Züchten der Zellen nach einem der Ansprüche 1 bis 6 in einem Medium, welches Lactat oder Lactat und verschiedene Arten von Hydroxyalkanoaten als Kohlenstoffquelle enthält; und Gewinnen von Polylactat oder dem Copolymer von Poly(3HB-Co-Lactat) aus den kultivierten Zellen.

8. Verfahren zur Herstellung von Copolymer[Poly(3HHx-Co-3HO-Co-3HD-Co-Lactat)] und Lactat, wobei das Verfahren umfasst: Züchten der Zellen nach Anspruch 1 in einem Medium, welches Lactat und Fettsäuren als Kohlenstoffquelle enthält; und Gewinnen von Copolymer[Poly(3HHx-Co-3HO-Co-3HD-Co-Lactat)] und Lactat aus den kultivierten Zellen, wobei die Fettsäure Decensäure ist.

9. Rekombinanter Vektor, welcher ein Propionyl-CoA-Transferase codierendes Gen und ein Polyhydroxyalkanoatsynthase codierendes Gen ausgewählt aus phaC und phaRBC enthält.

10. Rekombinanter Vektor nach Anspruch 9, wobei phaC aus *Pseudomonas sp.* stammt.

11. Zellen, die mit dem rekombinanten Vektor nach einem der Ansprüche 9 bis 10 transformiert sind.

12. Verfahren zur Herstellung von Polylactat oder einem Copolymer von Poly(3HB-Co-Lactat), wobei das Verfahren umfasst: Züchten der transformierten Zellen nach Anspruch 11 in einem Medium, welches Lactat oder Lactat und verschiedene Arten von Hydroxyalkanoaten als Kohlenstoffquelle enthält; und Gewinnen von Polylactat oder dem Copolymer aus Poly(3HB-Co-Lactat) aus den kultivierten Zellen.

## Revendications

1. Cellules transformées ayant la capacité de produire du polylactate ou un copolymère d'hydroxyalcanoate-lactate [poly(hydroxyalcanoate-co-lactate)], qui contiennent un gène codant pour la propionyl-CoA transférase et un gène codant pour la polyhydroxyalcanoate synthase choisi parmi phaC et phaRBC,
dans lesquelles lesdites cellules transformées sont obtenues en transformant des cellules n'ayant pas de gène codant pour la polyhydroxyalcanoate synthase choisi parmi phaC et phaRBC avec un vecteur recombinant contenant un gène codant pour la propionyl-CoA transférase et un gène codant pour la polyhydroxyalcanoate synthase choisi parmi phaC et phaRBC.

2. Cellules transformées ayant la capacité de produire du polylactate ou un copolymère d'hydroxyalcanoate-lactate [poly(hydroxyalcanoate-co-lactate)], qui contiennent un gène codant pour la propionyl-CoA transférase et un gène codant pour la polyhydroxyalcanoate synthase choisi parmi phaC et phaRBC,
dans lesquelles lesdites cellules transformées sont obtenues :
- par transformation des cellules ayant un gène codant pour la polyhydroxyalcanoate synthase choisi parmi phaC et phaRBC avec un vecteur recombinant contenant un gène codant pour la propionyl-CoA transférase ; ou
- par transformation des cellules ayant un gène codant pour la propionyl-CoA transférase avec un vecteur recombinant contenant un gène codant pour la polyhydroxyalcanoate synthase choisi parmi phaC et phaRBC.

3. Cellules transformées selon la revendication 1, dans lesquelles lesdites cellules sont transformées avec un vecteur recombinant contenant de la propionyl-CoA transférase, en même temps, transformées avec un vecteur contenant phaC ou phaRBC, ou phaC ou phaRBC sont insérés sur le chromosome de celui-ci.

4. Cellules transformées selon la revendication 1, dans lesquelles les cellules n'ayant pas de gène codant pour la polyhydroxyalcanoate synthase sont *E*. *coli.*

5. Cellules transformées selon la revendication 2, dans lesquelles lesdites cellules ayant un gène codant pour la propionyl-CoA transférase sont amplifiées avec ledit gène.

6. Cellules transformées selon la revendication 2, dans lesquelles lesdites cellules ayant un gène codant pour la propionyl-CoA transférase sont *Clostridium propionicum.*

7. Procédé de préparation de polylactate ou d'un copolymère de poly(3HB-co-lactate), le procédé comprenant : la culture des cellules selon l'une quelconque des revendications 1 à 6 dans un milieu contenant du lactate, ou du lactate et divers types d'hydroxyalcanoates comme source de carbone ; et la récupération du polylactate ou du copolymère de poly(3HB-co-lactate) à partir des cellules cultivées.

8. Procédé de préparation d'un copolymère de [poly(3HHx-co-3HO-co-3HD-co-lactate)] et de lactate, le procédé comprenant : la culture des cellules selon la revendication 1 dans un milieu contenant du lactate et un acide gras en tant que source de carbone ; et la récupération du copolymère de [poly(3HHx-co-3HO-co-3HD-co-lactate)] et du lactate à partir des cellules cultivées, dans lesquelles ledit acide gras est l'acide décénoïque.

9. Vecteur recombinant contenant un gène codant pour la propionyl-CoA transférase et un gène codant pour la polyhydroxyalcanoate synthase choisi parmi phaC et phaRBC.

10. Vecteur recombinant selon la revendication 9, dans lesquelles phaC est dérivé de *Pseudomonas sp.*

11. Cellules transformées avec le vecteur recombinant selon l'une quelconque des revendications 9 ou 10.

12. Procédé de préparation de polylactate ou d'un copolymère de poly(3HB-co-lactate), le procédé comprenant : la culture des cellules transformées selon la revendication 11 dans un milieu contenant du lactate ou du lactate et divers types d'hydroxyalcanoates comme source de carbone ; et la récupération du polylactate ou du copolymère de poly(3HB-co-lactate) à partir des cellules cultivées.


<110> LG CHEM, LTD.
   KOREA ADVANCED INSTITUTE OF SCIENCE AND TECHNOLOGY
<120> CELLS OR PLANTS HAVING A PRODUCING ABILITY OF POLYLACTATE OR ITS COPOLYMERS AND METHOD FOR PREPARING POLYLACTATE OR ITS COPOLYMERS USING THE SAME
<130> PP-B0253
<150> KR 10-2005-0043798
   <151> 2005-05-24
<160> 4
<170> KopatentIn 1.71
<210> 1
   <211> 39
   <212> DNA
   <213> Artificial Sequence
<220>
   <223> primer
<400> 1
   ggaattcatg agaaaggttc ccattattac cgcagatga 39
<210> 2
   <211> 46
   <212> DNA
   <213> Artificial Sequence
<220>
   <223> primer
<400> 2
   gctctagatt aggacttcat ttccttcaga cccattaagc cttctg 46
<210> 3
   <211> 27
   <212> DNA
   <213> Artificial Sequence
<220>
   <223> primer
<400> 3
   ggaattcatg aattgtttca aaaacga 27
<210> 4
   <211> 28
   <212> DNA
   <213> Artificial Sequence
<220>
   <223> primer
<400> 4
   cgggatcctt aattagaacg ctcttcaa 28